# EUROPEAN PATENT APPLICATION

(11) **EP 4 446 403 A1**
(43) Date of publication of application: **16.10.2024**
(21) Application number: 21967056.9
(22) Date of filing: 06.12.2021
(51) Int. Cl.: C12M 1/34, G01N 33/48, G01N 21/27, G02B 21/36

(54) **CLASSIFICATION DEVICE, CLASSIFICATION METHOD, AND PROGRAM**

(71) Applicant: NEC Corporation, 108-8001 Tokyo (JP)
(72) Inventor: OMI, Yasuo, Tokyo 108-8001 (JP)
(74) Representative: Betten & Resch
(86) International application number: PCT/JP2021/044623
(87) International publication number: WO 2023/105548

(57) **Abstract**

In order to improve accuracy in classification of a pathological sample between benignancy or malignancy, a classification apparatus (1) includes: an imaging section (11) for capturing an image which includes a partial range of a pathological sample as a subject; an acquisition section (12) for acquiring the image which has been captured by the imaging section (11); and a classification section (13) for classifying, as benign or malignant, a cell included as a subject in the image which has been acquired by the acquisition section (12).

## Description

### Technical Field

The present invention relates to a classification apparatus, a classification method, and a program for classifying a cell as benign or malignant.

### Background Art

A technique has been disclosed in which a sample is imaged using an imaging means such as a camera attached to a microscope, and a substance included as a subject in the sample is analyzed using the captured microscopic image.

For example, Patent Literature 1 discloses a method of carrying out analysis for classifying an image of a sample into a target region and a non-target region during a sample scanning operation using a microscope.

### Citation List

### [Patent Literature]

### [Patent Literature 1]

Published Japanese Translation of PCT International Application Tokuhyo No. 2010-503038

### Summary of Invention

### Technical Problem

In the technique of Patent Literature 1, in order to quickly generate a large amount of data, analysis of an image of a sample is carried out during the sample scanning operation. Therefore, in the technique of Patent Literature 1, in the technique of Patent Literature 1, there is room for improvement in terms of accuracy of analysis.

An example aspect of the present invention is accomplished in view of the above problem, and an example object thereof is to provide a technique for improving accuracy in classification of a pathological sample between benignancy and malignancy.

### Solution to Problem

A classification apparatus in accordance with an example aspect of the present invention includes: an imaging means for capturing an image which includes a partial range of a pathological sample as a subject; an acquisition means for acquiring the image which has been captured by the imaging means; and a classification means for classifying, as benign or malignant, a cell included as a subject in the image which has been acquired by the acquisition means.

A classification method in accordance with an example aspect of the present invention includes: capturing an image which includes a partial range of a pathological sample as a subject; acquiring the image which has been captured in the capturing; and classifying, as benign or malignant, a cell included as a subject in the image which has been acquired in the acquiring.

A program in accordance with an example aspect of the present invention is a program for causing a computer to function as a classification apparatus, the program causing the computer to function as: an imaging means for capturing an image which includes a partial range of a pathological sample as a subject; an acquisition means for acquiring the image which has been captured by the imaging means; and a classification means for classifying, as benign or malignant, a cell included as a subject in the image which has been acquired by the acquisition means.

### Advantageous Effects of Invention

According to an example aspect of the present invention, it is possible to improve accuracy in classification of a pathological sample between benignancy and malignancy.

### Brief Description of Drawings

Fig. 1 is a block diagram illustrating a configuration of a classification apparatus in accordance with a first example embodiment of the present invention.
Fig. 2 is a flowchart illustrating a flow of a classification method in accordance with the first example embodiment of the present invention.
Fig. 3 is a block diagram illustrating a configuration of a classification apparatus in accordance with a second example embodiment of the present invention.
Fig. 4 is a diagram illustrating an example in which an automatic control section causes an imaging range of an imaging section to move in the second example embodiment of the present invention.
Fig. 5 is a flowchart illustrating a flow of a classification method in accordance with the second example embodiment of the present invention.
Fig. 6 is a block diagram illustrating a configuration of a classification apparatus in accordance with a third example embodiment of the present invention.
Fig. 7 is a flowchart illustrating a flow of a classification method in accordance with the third example embodiment of the present invention.
Fig. 8 illustrates an example of an image which is displayed on a display apparatus in the third example embodiment of the present invention.
Fig. 9 is a block diagram illustrating a configuration of a classification apparatus in accordance with a fourth example embodiment of the present invention.
Fig. 10 is a flowchart illustrating a flow of a classification method in accordance with the fourth example embodiment of the present invention.
Fig. 11 illustrates an example of an image which is displayed on a display apparatus in the fourth example embodiment of the present invention.
Fig. 12 is a flowchart illustrating a flow of a classification method in accordance with a variation of the present invention.
Fig. 13 is a block diagram illustrating an example hardware configuration of the classification apparatus in accordance with the example embodiments of the present invention.

### Example Embodiments

### [First example embodiment]

The following description will discuss a first example embodiment of the present invention in detail, with reference to the drawings. The present example embodiment is a basic form of example embodiments described later.

### (Configuration of classification apparatus 1)

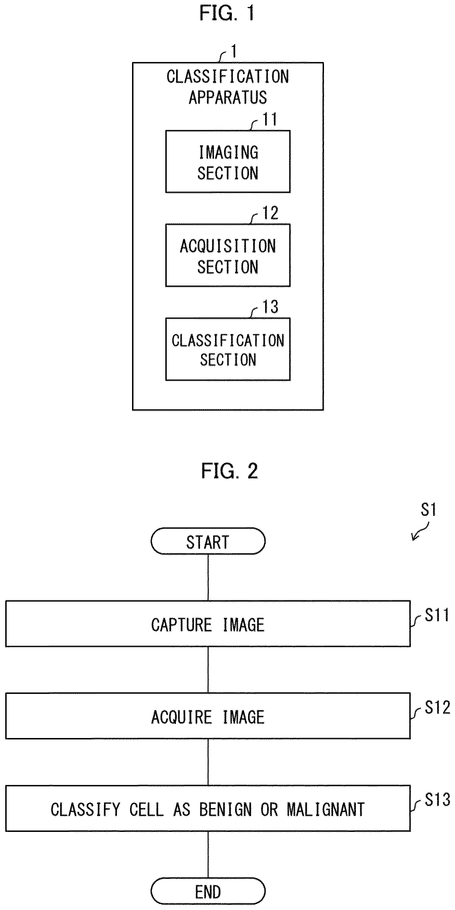

The following description will discuss a configuration of a classification apparatus 1 in accordance with the present example embodiment, with reference to Fig. 1. Fig. 1 is a block diagram illustrating a configuration of the classification apparatus 1 in accordance with the present example embodiment.

The classification apparatus 1 is an apparatus that acquires an image which includes a partial range of a pathological sample as a subject, and classifies, as benign or malignant, a cell included in the image as a subject.

The pathological sample is a sample which is diagnosed as benign or malignant in pathological diagnosis. The pathological sample includes also a sample that is used in cytodiagnosis in which a specimen cell included in the sample is classified as a benign cell or a malignant cell.

As illustrated in Fig. 1, the classification apparatus 1 includes an imaging section 11, an acquisition section 12, and a classification section 13. In the present example embodiment, the imaging section 11, the acquisition section 12, and the classification section 13 are components for implementing the imaging means, the acquisition means, and the classification means, respectively.

The imaging section 11 captures an image which includes a partial range of the pathological sample as a subject.

The acquisition section 12 acquires the image captured by the imaging section 11.

The classification section 13 classifies, as benign or malignant, a cell which is included as a subject in the image acquired by the acquisition section 12. A known method can be used as a method in which the classification section 13 classifies, as benign or malignant, the cell which is included as a subject in the image. For example, the classification section 13 is configured to input, into a trained model, the image acquired by the acquisition section 12, and acquire a classification result by the trained model, the trained model having been trained, while using as input an image which includes a cell as a subject, to classify the cell as benign or malignant.

As described above, the classification apparatus 1 in accordance with the present example embodiment employs the configuration of including: the imaging section 11 for capturing an image which includes a partial range of a pathological sample as a subject; the acquisition section 12 for acquiring the image which has been captured by the imaging section 11; and the classification section 13 for classifying, as benign or malignant, a cell included as a subject in the image which has been acquired by the acquisition section 12. Therefore, according to the classification apparatus 1 in accordance with the present example embodiment, it is possible to improve accuracy in classification of a pathological sample between benignancy and malignancy.

### (Flow of classification method S1)

The following description will discuss a flow of a classification method S1 in accordance with the present example embodiment, with reference to Fig. 2. Fig. 2 is a flowchart illustrating the flow of the classification method S1 in accordance with the present example embodiment.

### (Step S11)

In step S11, the imaging section 11 captures an image which includes a partial range of the pathological sample as a subject.

### (Step S12)

In step S12, the acquisition section 12 acquires the image captured by the imaging section 11 in step S11.

### (Step S13)

In step S13, the classification section 13 classifies, as benign or malignant, a cell which is included as a subject in the image which has been acquired by the acquisition section in step S12.

As described above, the classification method S1 in accordance with the present example embodiment employs the configuration in which: the imaging section 11 captures in step S11 an image which includes a partial range of a pathological sample as a subject; the acquisition section 12 acquires in step S12 the image which has been captured by the imaging section 11 in step S11; and the classification section 13 classifies in step S13, as benign or malignant, a cell included as a subject in the image which has been acquired by the acquisition section in step S12. Therefore, according to the classification method S1 in accordance with the present example embodiment, an example advantage similar to that of the classification apparatus 1 is brought about.

### [Second example embodiment]

The following description will discuss a second example embodiment of the present invention in detail, with reference to the drawings. The same reference numerals are given to constituent elements which have functions identical with those described in the foregoing example embodiment, and descriptions as to such constituent elements are omitted as appropriate.

### (Classification apparatus 2)

A classification apparatus 2 is an apparatus that classifies, as benign or malignant, a cell included in a pathological sample. More specifically, the classification apparatus 2 acquires a first image which includes a partial range of the pathological sample as a subject, and which has a first magnification as an imaging magnification. Next, in a case where the number of cells included as a subject in the first image is equal to or greater than a predetermined number (e.g., 10), the classification apparatus 2 acquires a second image at a second magnification, which is an imaging magnification higher than the first magnification. Then, the classification apparatus 2 classifies, as benign or malignant, a cell which is included as a subject in the acquired second image.

Here, processes from the process in which the classification apparatus 2 acquires the first image to the process in which the classification apparatus 2 classifies, as benign or malignant, a cell included as a subject in the second image are referred to as an automatic classification process.

In a case where the number of cells which have been classified as malignant is less than the predetermined number, the classification apparatus 2 moves the imaging range, and carries out the automatic classification process again.

The first magnification is not particularly limited, and is preferably a magnification which is sufficient to detect cells which are included as a subject in a pathological sample and to count the number of cells. For example, in the present example embodiment, the first magnification is 10 times.

It is preferable that the second magnification is a magnification which is higher than the first magnification and is sufficient to classify, as benign or malignant, a cell included in a pathological sample as a subject. For example, in the present example embodiment, the second magnification is 40 times.

### (Configuration of classification apparatus 2)

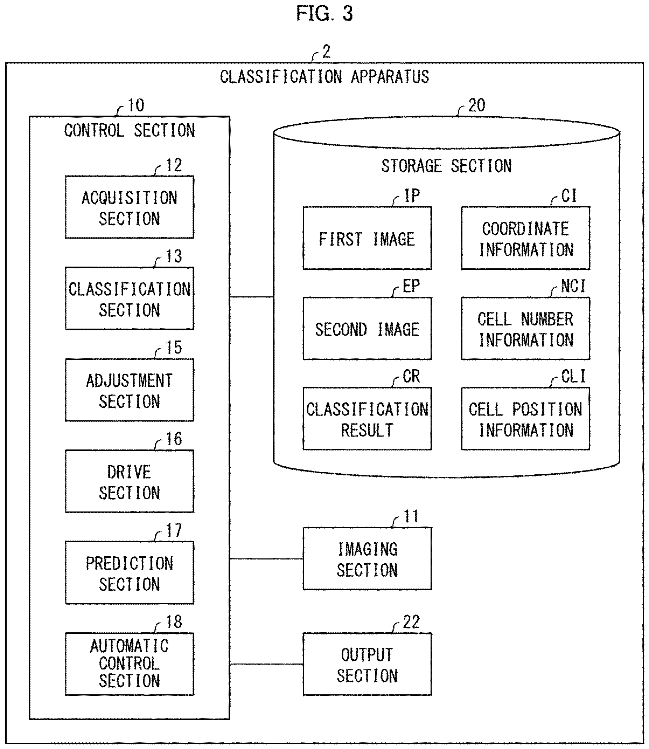

The following description will discuss a configuration of a classification apparatus 2 in accordance with the present example embodiment, with reference to Fig. 3. Fig. 3 is a block diagram illustrating a configuration of the classification apparatus 2 in accordance with the present example embodiment.

As illustrated in Fig. 3, the classification apparatus 2 includes a control section 10, an imaging section 11, a storage section 20, and an output section 22. In the present example embodiment, the imaging section 11 is a component for implementing the imaging means.

The imaging section 11 captures an image which includes a partial range of the pathological sample as a subject. The imaging section 11 includes, for example, an imaging sensor, and captures an image in which a target included in an angle of view appears as a subject. The imaging magnification of the imaging section 11 is adjusted by an adjustment section 15 (described later). For example, the imaging magnification of the imaging section is adjusted to a first magnification or a second magnification. The imaging section 11 supplies a first image IP which has been captured at the first magnification and a second image EP which has been captured at the second magnification to the control section 10 (described later).

In the storage section 20, data referred to by the control section 10 (described later) is stored. Examples of data stored in the storage section 20 include a first image IP, a second image EP, coordinate information CI, cell number information NCI, and cell position information CLI. The coordinate information CI, the cell number information NCI, and the cell position information CLI will be described later.

The output section 22 is an interface that outputs data supplied from the control section 10 (described later) to another apparatus connected thereto. Examples of another apparatus connected thereto include a display apparatus for displaying an image and a speaker for outputting audio.

### (Control section 10)

The control section 10 controls constituent elements of the classification apparatus 2. For example, the control section 10 causes the storage section 20 to store data and supplies data to the output section 22.

As illustrated in Fig. 3, the control section 10 functions also as an acquisition section 12, a classification section 13, an adjustment section 15, a drive section 16, a prediction section 17, and an automatic control section 18. In the present example embodiment, the acquisition section 12, the classification section 13, the adjustment section 15, the drive section 16, the prediction section 17, and the automatic control section 18 are components for implementing the acquisition means, the classification means, the adjustment means, the drive means, the prediction means, and the control means, respectively.

The acquisition section 12 acquires an image captured by the imaging section 11. For example, the acquisition section 12 acquires, in accordance with an instruction from the automatic control section 18 (described later), a first image IP and a second image EP which have been captured by the imaging section 11. The acquisition section 12 causes the storage section 20 to store the acquired first image IP and second image EP.

The classification section 13 acquires the second image EP which is stored in the storage section 20, and classifies, as benign or malignant, a cell included as a subject in the second image EP. The classification section 13 causes the storage section 20 to store a classification result CR indicating a result of the classification. For example, the classification section 13 carries out a classification process in accordance with an instruction from the automatic control section 18 (described later). A method in which the classification section 13 classifies, as benign or malignant, a cell included as a subject in the second image EP is as described above.

The adjustment section 15 adjusts an imaging magnification of the imaging section 11. For example, the adjustment section 15 adjusts the imaging magnification of the imaging section 11 to a first magnification or a second magnification in accordance with an instruction from the automatic control section 18 (described later).

The drive section 16 moves the imaging range of the imaging section 11. For example, the drive section 16 moves, in accordance with an instruction from the automatic control section 18 (described later), the imaging range so that the imaging range exhaustively moves over a whole of the pathological sample from a center of the pathological sample. With this configuration, the classification apparatus 2 can start the classification process from the center of the pathological sample where many cells are considered to be included. Therefore, it is possible to quickly carry out the classification process.

The prediction section 17 predicts the number of cells included as a subject in the image. For example, the prediction section 17 detects, in accordance with an instruction from the automatic control section 18 (described later), cells included as a subject in the first image IP which has been acquired by the acquisition section 12, and predicts the number of detected cells. A method in which the prediction section 17 detects cells and predicts the number of cells can be a known method as described below.
Step 1: A regressor which has been trained to convert a pathological image from RGB gradation into grayscale gradation according to staining intensity (an image after this gradation conversion is referred to as "staining intensity image") is prepared.
Step 2: Using the above regressor, an image to be analyzed is converted into a staining intensity image.
Step 3: A region which has a certain staining intensity or higher and has a certain circularity or higher is extracted from the staining intensity image.

As another example, the prediction section 17 is configured so as to (i) input a first image IP into a trained model which has been trained, while using as input an image which includes a cell as a subject, to detect cells included in the image and calculate the number of detected cells, and (ii) acquire the number of cells which is output by the trained model. The prediction section 17 causes the storage section 20 to store cell number information NCI indicating the predicted number of cells.

Moreover, the prediction section 17 causes the storage section 20 to store cell position information CLI indicating a position of the detected cell. For example, the prediction section 17 sets a center of the first image IP to be a center of a two-dimensional coordinate system, and generates cell position information CLI indicating coordinates of the position of the detected cell.

### (Process carried out by automatic control section 18)

The automatic control section 18 controls the acquisition section 12, the classification section 13, the adjustment section 15, and the drive section 16. For example, the automatic control section 18 provides instructions to the sections in order to carry out the following automatic classification process.
(1) The adjustment section 15 is instructed to adjust the imaging magnification of the imaging section 11 to a first magnification.
(2) The acquisition section 12 is instructed to acquire a first image IP which has been captured by the imaging section 11.
(3) The prediction section 17 is instructed to detect cells included as a subject in the first image IP, and to predict the number of detected cells.
(4) In a case where the number of cells predicted by the prediction section 17 is equal to or greater than a predetermined number, the adjustment section 15 is instructed to adjust the imaging magnification of the imaging section 11 to a second magnification, the acquisition section 12 is instructed to acquire a second image EP, and the classification section 13 is instructed to classify, as benign or malignant, a cell which is included as a subject in the second image EP.

In a case where the number of cells which have been classified as malignant by the classification section 13 is less than the predetermined number as a result of the automatic classification process, the automatic control section 18 instructs the drive section 16 to move the imaging range, and carries out the automatic classification process again.

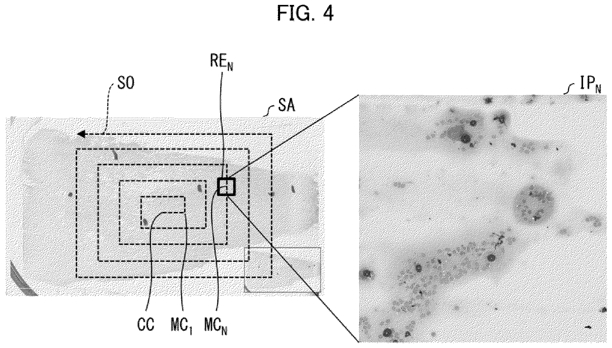

For example, the automatic control section 18 sets a center of the pathological sample to be a center of a two-dimensional coordinate system, and generates coordinate information CI indicating coordinates of a movement destination of the drive section 16. Then, the automatic control section 18 instructs the drive section 16 to move the imaging range of the imaging section 11 by supplying the generated coordinate information CI to the drive section 16. The automatic control section 18 causes the storage section 20 to store the generated coordinate information CI. The following description will discuss an example in which the automatic control section 18 causes the imaging range of the imaging section 11 to move, with reference to Fig. 4.

### (Example of movement of imaging range)

Fig. 4 is a diagram illustrating an example in which the automatic control section 18 causes the imaging range of the imaging section 11 to move in the present example embodiment.

The left part of Fig. 4 is a diagram of a pathological sample SA. As illustrated in the left part of Fig. 4, the automatic control section 18 sets a center of the pathological sample to be a center CC of a two-dimensional coordinate system. In the example illustrated in Fig. 4, the automatic control section 18 causes the imaging section 11 to move as in a scanning order SO in which the imaging section 11 is exhaustively moved over a whole of the pathological sample from the center CC of the pathological sample.

The automatic control section 18 first generates coordinate information CI indicating coordinates of the center CC in order to cause the imaging section 11 to move to the center CC of the pathological sample SA. Next, the automatic control section 18 supplies the generated coordinate information CI to the drive section 16, and thus provides an instruction to the drive section 16 such that a center the imaging range of the imaging section 11 comes to the center CC. The automatic control section 18 causes the storage section 20 to store the generated coordinate information CI. When the drive section 16 has moved the imaging section 11 to a position where the center of the imaging range of the imaging section 11 is at the center CC, the automatic control section 18 carries out the automatic classification process.

In a case where the number of cells which have been classified as malignant by the classification section 13 is less than the predetermined number as a result of the automatic classification process, the automatic control section 18 refers to, in order to move the imaging range, the coordinate information CI stored in the storage section 20. The automatic control section 18 refers to the coordinate information CI and the scan order SO, and generates coordinate information CI indicating coordinates of a position MC₁ which is a next movement destination of the imaging section 11. The automatic control section 18 instructs the drive section 16 to set the center of the imaging range of the imaging section 11 to be the position MC₁ by supplying the generated coordinate information CI to the drive section 16. The automatic control section 18 causes the storage section 20 to store the generated coordinate information CI. When the drive section 16 has moved the imaging section 11 so that the center of the imaging range of the imaging section 11 is at the position MC₁, the automatic control section 18 carries out the automatic classification process.

Thus, in a case where the number of cells which have been classified as malignant by the classification section 13 is less than the predetermined number as a result of the automatic classification process, the automatic control section 18 causes the imaging section 11 to move as in the scanning order SO in which the imaging section 11 is exhaustively moved over the whole of the pathological sample from the center CC of the pathological sample. For example, in a case where the automatic control section 18 has caused the imaging section 11 to move such that the center of the imaging range of the imaging section 11 is at a position MC_{N}, the automatic control section 18 instructs the acquisition section 12 to acquire a first image IP_{N} of an imaging range RE_{N} (see the right part of Fig. 4), and starts the automatic classification process.

### (Flow of classification method S2)

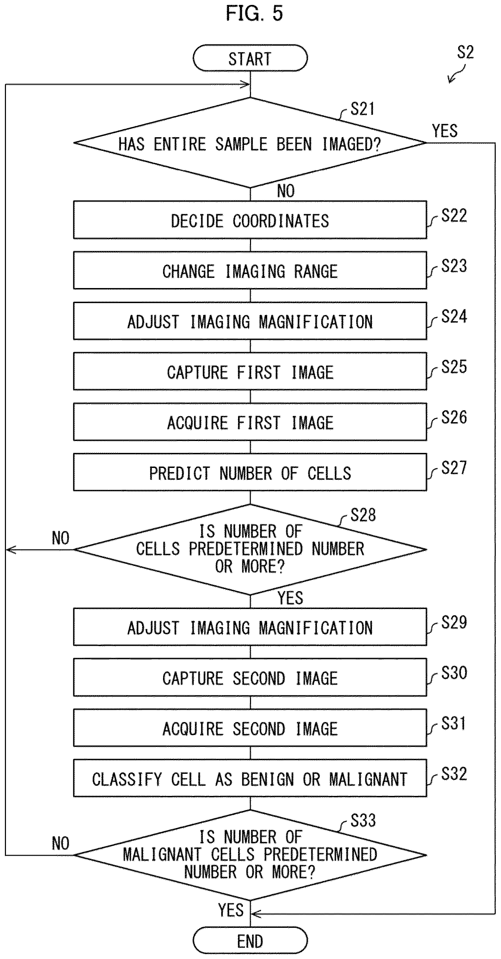

The following description will discuss a flow of a classification method S2 which is carried out by the classification apparatus 2 in accordance with the present example embodiment, with reference to Fig. 5. Fig. 5 is a flowchart illustrating the flow of the classification method S2 in accordance with the present example embodiment.

### (Step S21)

In step S21, the automatic control section 18 refers to coordinate information CI stored in the storage section 20, and determines whether or not the imaging section 11 has been moved to the end of the scanning order SO.

In a case where it has been determined in step S21 that the entire sample has been imaged (step S21: Yes), the classification apparatus 2 ends the process of Fig. 5.

### (Step S22)

In a case where it has been determined in step S21 that the entire sample has not been imaged (step S21: No), the automatic control section 18 decides in step S22 coordinates of a movement destination of the imaging section 11. The automatic control section 18 generates coordinate information CI indicating the coordinates of the movement destination, and supplies the generated coordinate information CI to the drive section 16. The automatic control section 18 causes the storage section 20 to store the generated coordinate information CI.

### (Step S23)

In step S23, the drive section 16 moves the imaging section 11 to a position of the coordinates indicated by the coordinate information CI which has been supplied from the automatic control section 18 in step S22.

### (Step S24)

In step S24, the automatic control section 18 instructs the adjustment section 15 to adjust the imaging magnification of the imaging section 11 to the first magnification. The adjustment section 15 adjusts the imaging magnification of the imaging section 11 to the first magnification.

### (Step S25)

In step S25, the imaging section 11 captures a first image IP.

### (Step S26)

In step S26, the automatic control section 18 instructs the acquisition section 12 to acquire the first image IP which has been captured by the imaging section 11. The acquisition section 12 acquires the first image IP which has been captured by the imaging section 11. The acquisition section 12 causes the storage section 20 to store the acquired first image IP.

### (Step S27)

In step S27, the automatic control section 18 instructs the prediction section 17 to predict the number of cells included as a subject in the first image IP. The prediction section 17 acquires the image IP which is stored in the storage section 20, and detects cells included as a subject in the image IP. Then, the prediction section 17 predicts the number of detected cells. Moreover, the prediction section 17 causes the storage section 20 to store cell number information NCI indicating the predicted number of cells and cell position information CLI indicating a position of the detected cell.

### (Step S28)

In step S28, the automatic control section 18 acquires the cell number information NCI which is stored in the storage section 20, and determines whether or not the number of cells indicated by the cell number information NCI is equal to or greater than a predetermined number.

In a case where it has been determined in step S28 that the number of cells is less than the predetermined number (step S28: No), the classification apparatus 2 returns to the process of step S21.

### (Step S29)

In a case where it has been determined in step S28 that the number of cells is equal to or greater than the predetermined number (step S28: Yes), the automatic control section 18 instructs, in step S29, the adjustment section 15 to adjust the imaging magnification of the imaging section 11 to the second magnification. The adjustment section 15 adjusts the imaging magnification of the imaging section 11 to the second magnification.

### (Step S30)

In step S30, the imaging section 11 captures a second image EP.

As an example of a method of capturing a second image EP, the automatic control section 18 acquires cell position information CLI which is stored in the storage section 20, and causes the imaging section 11 to move to a position indicated by the position information via the drive section 16 so that a position of the cell indicated by the cell position information CLI comes to the center of the imaging range. Then, the imaging section 11 captures a second image EP.

For example, in a case where the cell number information NCI stored in the storage section 20 indicates that the predicted number of cells is N, it is preferable to capture N second images EP in which respective positions of N cells are each at the center of the imaging range.

### (Step S31)

In step S31, the automatic control section 18 instructs the acquisition section 12 to acquire the second image EP which has been captured by the imaging section 11. The acquisition section 12 acquires the second image EP which has been captured by the imaging section 11. The acquisition section 12 causes the storage section 20 to store the acquired second image EP.

### (Step S32)

In step S32, the automatic control section 18 instructs the classification section 13 to carry out a classification process. The classification section 13 acquires the second image EP which is stored in the storage section 20, and classifies, as benign or malignant, a cell included as a subject in the second image EP. The classification section 13 causes the storage section 20 to store a classification result CR indicating a result of the classification.

### (Step S33)

In step S33, the automatic control section 18 acquires the classification result CR which is stored in the storage section 20, and determines whether or not the number of cells which have been classified as malignant by the classification section 13 is equal to or greater than the predetermined number.

In a case where it has been determined in step S33 that the number of cells which have been classified as malignant is equal to or greater than the predetermined number (step S33: Yes), the classification apparatus 2 ends the process of Fig. 5.

In a case where it has been determined in step S33 that the number of cells which have been classified as malignant is less than the predetermined number (step S33: No), the classification apparatus 2 returns to the process of step S21.

As described above, in a case where the number of cells included as a subject in the first image IP which has been captured at the first imaging magnification is equal to or greater than a predetermined number, the classification apparatus 2 in accordance with the present example embodiment acquires the second image EP which has been captured at the second imaging magnification which is higher than the first imaging magnification. Further, the classification apparatus 2 in accordance with the present example embodiment employs the configuration in which a cell included as a subject in the second image EP is classified as benign or malignant.

Therefore, according to the classification apparatus 2 in accordance with the present example embodiment, a cell included as a subject in the second captured image EP, which has been obtained by enlarging a region in which the number of cells included is equal to or greater than the predetermined number, is classified as benign or malignant. Therefore, it is possible to improve accuracy in classification of a pathological sample between benignancy and malignancy.

According to the classification apparatus 2 in accordance with the present example embodiment, the imaging range is automatically changed, and the automatic classification process is carried out in the changed imaging range. Therefore, according to the classification apparatus 2 in accordance with the present example embodiment, it is possible to obtain the same classification result regardless of a skill of a user who uses the classification apparatus 2.

### [Third example embodiment]

The following description will discuss a third example embodiment of the present invention in detail, with reference to the drawings. The same reference numerals are given to constituent elements which have functions identical with those described in the foregoing example embodiments, and descriptions as to such constituent elements are omitted as appropriate.

### (Classification apparatus 3)

In a case where, in step S27 of the flowchart illustrated in Fig. 5 above, the number of cells predicted by the prediction section 17 is 1 or more (in other words, a cell has been detected as a subject in a first image IP), the classification apparatus 3 in accordance with the present example embodiment displays, according to an instruction from a user, a second image EP which has been captured so that a cell included as a subject in the first image IP appears at the center of the imaging range. The instruction from the user received by the classification apparatus 3 will be described later.

### (Configuration of classification apparatus 3)

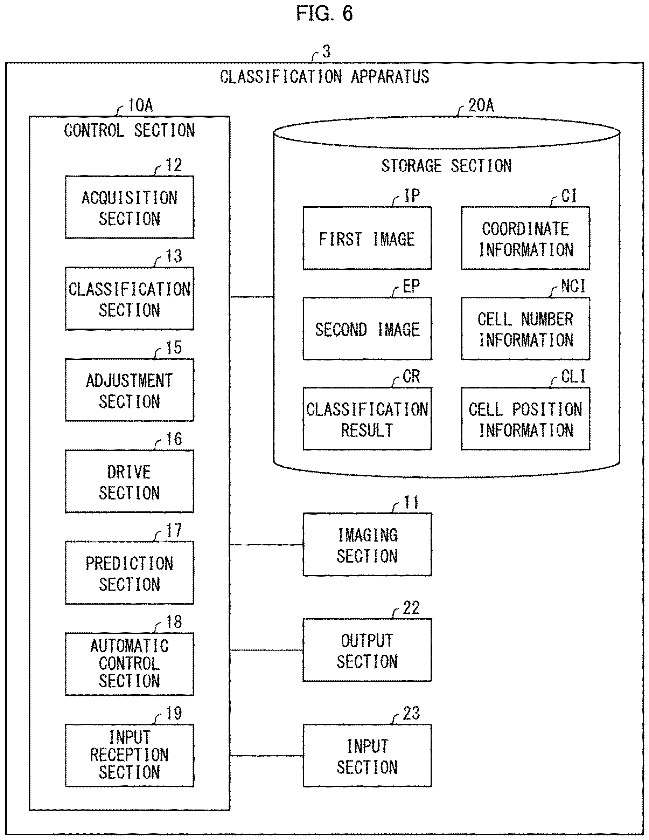

The following description will discuss a configuration of the classification apparatus 3 in accordance with the present example embodiment, with reference to Fig. 6. Fig. 6 is a block diagram illustrating a configuration of the classification apparatus 3 in accordance with the present example embodiment.

As illustrated in Fig. 6, the classification apparatus 3 includes a control section 10A, an imaging section 11, a storage section 20A, an output section 22, and an input section 23. In the present example embodiment, the imaging section 11 is a component for implementing the imaging means.

The imaging section 11 is as described above.

In the storage section 20A, cell position information CLI indicating a position of a detected cell in the data stored in the storage section 20 described above is stored in association with a classification result CR.

The output section 22 is an interface that outputs data supplied from the control section 10A (described later) to another apparatus connected thereto. In the present example embodiment, the output section 22 acquires image data from the control section 10A, and outputs the image data to the display apparatus connected thereto.

The input section 23 is an interface that receives operation from a user. The input section 23 supplies instruction information indicating the operation received from the user to the control section 10A.

### (Control section 10A)

The control section 10A controls constituent elements of the classification apparatus 3. For example, the control section 10A causes the storage section 20A to store data and supplies data to the output section 22.

As illustrated in Fig. 6, the control section 10A functions also as an acquisition section 12, a classification section 13, an adjustment section 15, a drive section 16, a prediction section 17, an automatic control section 18, and an input reception section 19. In the present example embodiment, the acquisition section 12, the classification section 13, the adjustment section 15, the drive section 16, the prediction section 17, the automatic control section 18, and the input reception section 19 function also as the acquisition means, the classification means, the adjustment means, the drive means, the prediction means, the control means, and the reception means.

The acquisition section 12, the adjustment section 15, and the drive section 16 are as described above.

The prediction section 17 predicts the number of cells included as a subject in the image. For example, the prediction section 17 detects, in accordance with an instruction from the automatic control section 18 (described later), cells included as a subject in a first image IP which has been acquired by the acquisition section 12, and predicts the number of detected cells. As described above, a known method can be used as a method in which the prediction section 17 predicts the number of cells. The prediction section 17 causes the storage section 20A to store cell number information NCI indicating the predicted number of cells and cell position information CLI indicating a position of the detected cell. For example, the prediction section 17 sets a center of a second image EP to be a center of a two-dimensional coordinate system, and generates cell position information CLI indicating coordinates of the position of the detected cell.

The classification section 13 acquires the second image EP and the cell position information CLI which are stored in the storage section 20A, and classifies, as benign or malignant, a cell which is included as a subject in the second image EP and which is at a position indicated by the cell position information CLI. For example, the classification section 13 carries out a classification process in accordance with an instruction from the automatic control section 18 (described later). A method in which the classification section 13 classifies, as benign or malignant, a cell included as a subject in the second image EP is as described above. The classification section 13 causes the storage section 20A to store the cell position information CLI indicating the position of the classified cell and the classification result CR indicating the result of classification in association with each other.

The input reception section 19 receives an instruction from a user. Specifically, the input reception section 19 acquires instruction information which indicates operation received from the user and which has been supplied from the input section 23. The input reception section 19 supplies the acquired instruction information to the automatic control section 18.

### (Process carried out by automatic control section 18 in present example embodiment)

In a case where the number of cells predicted by the prediction section 17 is 1 or more, the automatic control section 18, in addition to the process in the foregoing example embodiments, instructs the drive section 16 to move the imaging range such that any of cells detected by the prediction section 17 comes to the center, and carries out an automatic display process in which the display apparatus is caused to display the image acquired by the acquisition section 12.

The automatic control section 18 can be configured to carry out or not to carry out the automatic display process depending on operation from a user. For example, the automatic control section 18 displays a button (e.g., a "display detail" button) for receiving an instruction to display a second image EP in which any of detected cells is included at the center of the imaging range as a subject. In a case where the instruction information acquired by the input reception section 19 indicates that the button has been pressed, the automatic control section 18 carries out the automatic display process.

For the automatic display process, more specifically, the automatic control section 18 refers to the cell position information CLI which is stored in the storage section 20A, and instructs the drive section 16 to move the imaging section 11 so that a position indicated by the cell position information CLI comes to the center of the imaging range. The drive section 16 moves the imaging section 11 in accordance with the instruction from the automatic control section 18.

The automatic control section 18 instructs the adjustment section 15 to adjust the imaging magnification to the second magnification. The adjustment section 15 adjusts the imaging magnification of the imaging section 11 to the second magnification in accordance with the instruction from the automatic control section 18.

Then, the automatic control section 18 instructs the acquisition section 12 to acquire the second image EP. The acquisition section 12 acquires, in accordance with the instruction from the automatic control section 18, the second image EP which has been captured by the imaging section 11 after the movement and after the imaging magnification adjustment. The automatic control section 18 causes, via the output section 22, the display apparatus to display image data indicating the second image EP.

### (Flow of classification method S3)

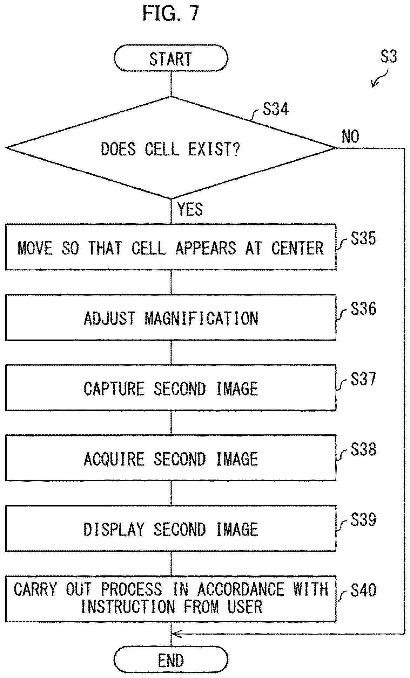

The following description will discuss a flow of a classification method S3 which is carried out by the classification apparatus 3 in accordance with the present example embodiment, with reference to Fig. 7. Fig. 7 is a flowchart illustrating the flow of the classification method S3 in accordance with the present example embodiment. For example, the flowchart illustrated in Fig. 7 is carried out after the process of step S32 in the foregoing classification method S2. For example, the flowchart illustrated in Fig. 7 is carried out in a case where an instruction has been received, from the user, to display a second image EP in which any of detected cells is included as a subject at the center of the imaging range.

### (Step S34)

In step S34, the automatic control section 18 acquires cell number information NCI from the storage section 20A, and determines whether or not a cell exists in the imaging range.

In step S34, in a case where it has been determined that no cell exists in the imaging range (step S34: No), the classification apparatus 3 ends the process of Fig. 7.

### (Step S35)

In a case where it has been determined in step S34 that a cell exists in the imaging range (step S34: Yes), the automatic control section 18 refers to cell position information CLI which is stored in the storage section 20A in step S35, and instructs the drive section 16 to move the imaging range of the imaging section 11 so that a position of a cell indicated by the cell position information CLI comes to the center of the imaging range of the imaging section 11.

### (Step S36)

In step S36, the automatic control section 18 instructs the adjustment section 15 to adjust the imaging magnification of the imaging section 11 to the second magnification. The adjustment section 15 adjusts the imaging magnification of the imaging section 11 to the second magnification.

### (Step S37)

In step S37, the imaging section 11 captures a second image EP in which the cell is included as a subject at the center of the imaging range.

### (Step S38)

In step S38, the automatic control section 18 instructs the acquisition section 12 to acquire the second image EP which has been captured by the imaging section 11. The acquisition section 12 acquires the second image EP which has been captured by the imaging section 11. The acquisition section 12 causes the storage section 20A to store the acquired second image EP.

### (Step S39)

In step S38, the automatic control section 18 acquires the second image EP from the storage section 20A, and causes, via the output section 22, the display apparatus to display the acquired second image EP.

### (Step S40)

In step S40, the automatic control section 18 carries out the automatic display process indicated in steps S35 through S39, and then carries out a process in accordance with the instruction received by the input reception section 19.

For example, in a case where the input reception section 19 has acquired instruction information indicating that an automatic display process is carried out so that another cell appears at the center of the imaging range, the automatic control section 18 carries out the automatic display process indicated in steps S35 through S39, and then carries out the automatic display process in accordance with the instruction received by the input reception section 19 so that another cell appears at the center of the imaging range.

For example, the automatic control section 18 superimposes and displays, on the second image EP which is displayed in step S39, a button (e.g., a "next" button) for receiving an instruction from the user to display the second image EP in which another cell is included as a subject at the center of the imaging range. Then, in a case where the input reception section 19 has acquired instruction information indicating that the button has been pressed, the automatic control section 18 refers to cell position information CLI which is stored in the storage section 20A, and carries out the automatic display process so that another cell is displayed at the center of the imaging range.

As another example, in a case where the instruction from the user received by the input reception section 19 is an instruction to carry out the automatic display process so that another cell appears at the center of the imaging range each time a predetermined time period elapses, the automatic control section 18 carries out the automatic display process so that another cell appears at the center of the imaging range each time the predetermined time period elapses.

For example, the automatic control section 18 superimposes and displays, on the second image EP which is displayed in step S39, a button (e.g., an "automatic display" button) for receiving an instruction from the user to automatically display, each time a predetermined time period (e.g., 10 seconds) elapses, the second image EP in which another cell is included as a subject at the center of the imaging range. Then, in a case where the input reception section 19 has acquired instruction information indicating that the button has been pressed, the automatic control section 18 refers to cell position information CLI which is stored in the storage section 20A, and carries out the automatic display process so that another cell appears at the center of the imaging range each time the predetermined time period elapses.

As another example, in a case where the instruction from the user received by the input reception section 19 is an instruction to display only a cell which has been classified as malignant, the automatic control section 18 carries out the automatic display process so that a cell which has been classified as malignant appears at the center of the imaging range. In other words, the automatic control section 18 does not carry out an automatic display process in which a cell which has been classified as benign appears at the center of the imaging range.

For example, the automatic control section 18 superimposes and displays, on the second image EP which is displayed in step S39, a button (e.g., a "display malignant cell only" button) for receiving an instruction from a user to display only a cell which has been classified as malignant. Then, in a case where the input reception section 19 has acquired instruction information indicating that the button has been pressed, the automatic control section 18 refers to the classification result CR which is stored in the storage section 20A. In a case where the classification result CR indicates that the cell has been classified as malignant, the automatic control section 18 refers to cell position information CLI associated with the classification result CR. Then, the automatic control section 18 instructs the drive section 16 to move the imaging range such that the cell which has been classified as malignant by the classification section 13 appears at the center. Furthermore, the automatic control section 18 carries out the automatic display process of causing the display apparatus to display the second image EP which has been acquired by the acquisition section 12.

### (Example 1 of displayed image)

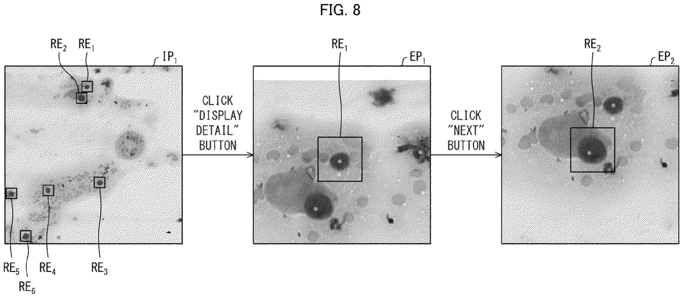

The following description will discuss an example of an image displayed on a display apparatus in the present example embodiment, with reference to Fig. 8. Fig. 8 illustrates an example of an image which is displayed on a display apparatus in the present example embodiment.

The left part of Fig. 8 is a first image IP₁ which includes a plurality of cells as a subject, and has been captured at the first magnification. The first image IP₁ includes regions RE₁ through RE₆ which include the respective plurality of cells. Here, in a case where the input reception section 19 has acquired instruction information indicating that a button (e.g., a "display detail" button), which is a button for receiving an instruction to display a second image EP in which any of detected cells is included as a subject at the center of the imaging range, has been pressed, the automatic control section 18 displays the second image EP in which any of the cells is included as a subject at the center of the imaging range. The automatic control section 18 displays the second image EP at the second magnification, which is a magnification larger than the first magnification.

The middle part of Fig. 8 is a second image EP₁ which has been captured so that the cell included in the region RE₁ appears at the center of the imaging range. Here, in a case where the input reception section 19 has acquired instruction information indicating that a button (e.g., a "next" button), which is a button for receiving from a user an instruction to display a second image EP in which another cell is included as a subject at the center of the imaging range, has been pressed, the automatic control section 18 displays the second image EP in which another cell is included as a subject at the center of the imaging range.

The right part of Fig. 8 is a second image EP₂ which has been captured so that another cell included in the region RE₂ appears at the center.

As described above, the classification apparatus 3 in accordance with the present example embodiment employs the configuration in which a second image EP, which has been captured so that a cell included as a subject in a first image IP appears at the center of the imaging range, is automatically displayed. Therefore, the classification apparatus 3 in accordance with the present example embodiment can allow the user to check whether or not the classification result is correct. A cell is automatically displayed at the center in the second image EP, and it is therefore possible to facilitate checking work by the user.

### [Fourth example embodiment]

The following description will discuss a fourth example embodiment of the present invention in detail, with reference to the drawings. The same reference numerals are given to constituent elements which have functions identical with those described in the foregoing example embodiments, and descriptions as to such constituent elements are omitted as appropriate.

### (Classification apparatus 4)

A classification apparatus 4 is an apparatus that classifies, as benign or malignant, a cell included in a pathological sample. More specifically, in the classification apparatus 4, an imaging range in which a pathological sample is imaged at a first imaging magnification is changed by a user. In a case where operation to move the imaging range has not been carried out for a predetermined time period, the classification apparatus 4 acquires a second image EP at a second imaging magnification, which is higher than the first imaging magnification. Then, the classification apparatus 4 classifies, as benign or malignant, a cell which is included as a subject in the acquired second image EP.

### (Configuration of classification apparatus 4)

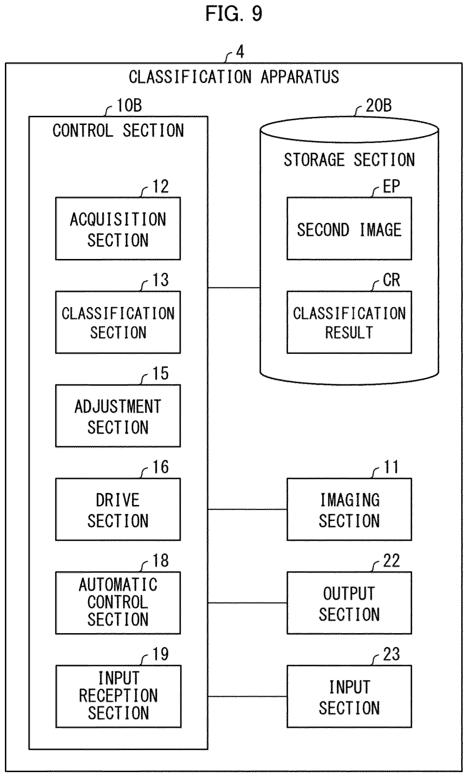

The following description will discuss a configuration of the classification apparatus 4 in accordance with the present example embodiment, with reference to Fig. 9. Fig. 9 is a block diagram illustrating a configuration of the classification apparatus 4 in accordance with the present example embodiment.

As illustrated in Fig. 9, the classification apparatus 4 includes a control section 10B, an imaging section 11, a storage section 20B, an output section 22, and an input section 23. In the present example embodiment, the imaging section 11 is a component for implementing the imaging means. The imaging section 11, the output section 22, and the input section 23 are as described above.

The storage section 20B stores, for example, the above described second image EP and a classification result CR.

### (Control section 10B)

The control section 10B controls constituent elements of the classification apparatus 4. For example, the control section 10B causes the storage section 20B to store data and supplies data to the output section 22.

As illustrated in Fig. 9, the control section 10B includes an acquisition section 12, a classification section 13, an adjustment section 15, a drive section 16, an automatic control section 18, and an input reception section 19. In the present example embodiment, the acquisition section 12, the classification section 13, the adjustment section 15, the drive section 16, the automatic control section 18, and the input reception section 19 are components for implementing the acquisition means, the classification means, the adjustment means, the drive means, the control means, and the reception means, respectively. The acquisition section 12 and the classification section 13 are as described above.

The adjustment section 15 adjusts an imaging magnification of the imaging section 11. For example, the adjustment section 15 adjusts the imaging magnification of the imaging section 11 to a first magnification or a second magnification in accordance with an instruction from the automatic control section 18 (described later).

The drive section 16 moves the imaging range of the imaging section 11. For example, the drive section 16 moves the imaging range of the imaging section 11 in accordance with an instruction from the automatic control section 18 (described later).

The input reception section 19 receives an instruction from a user. Specifically, in a case where the input section 23 has received, from the user, operation to move the imaging range of the imaging section 11 and operation to change the imaging magnification of the imaging section 11, the input reception section 19 acquires instruction information indicating the operation received by the input section 23. The input reception section 19 supplies the acquired instruction information to the automatic control section 18.

The automatic control section 18, in addition to the foregoing functions, acquires the instruction information which has been supplied from the input reception section 19. The automatic control section 18 instructs the drive section 16 to move the imaging range in accordance with an instruction from the user which is indicated in the acquired instruction information. Moreover, the automatic control section 18 instructs the adjustment section 15 to adjust the imaging magnification in accordance with an instruction from the user which is indicated in the acquired instruction information.

For example, in a case where the instruction information indicates an instruction to change the imaging range of the imaging section 11, the automatic control section 18 instructs the drive section 16 to move the imaging range. In a case where the instruction information indicates an instruction to adjust the imaging magnification from the first magnification to the second magnification, the automatic control section 18 instructs the adjustment section 15 to adjust the imaging magnification from the first magnification to the second magnification.

The automatic control section 18 determines whether or not the input reception section 19 has acquired, from the input section 23, input information indicating operation by the user within a predetermined time period (e.g., 10 seconds). For example, the automatic control section 18 determines whether or not input information indicating operation to move the imaging range has been further acquired from the input reception section 19 within a predetermined time period from when input information indicating operation to move the imaging range has been acquired.

For example, in a case where the input reception section 19 has not received, for a predetermined time period, an instruction to cause the drive section 16 to move the imaging range, the automatic control section 18 instructs the adjustment section 15 to adjust the imaging magnification to the second magnification, instructs the acquisition section 12 to acquire a second image EP, and carries out an automatic classification process in which the classification section 13 is instructed to classify, as benign or malignant, a cell which is included as a subject in the second image EP.

### (Flow of classification method S4)

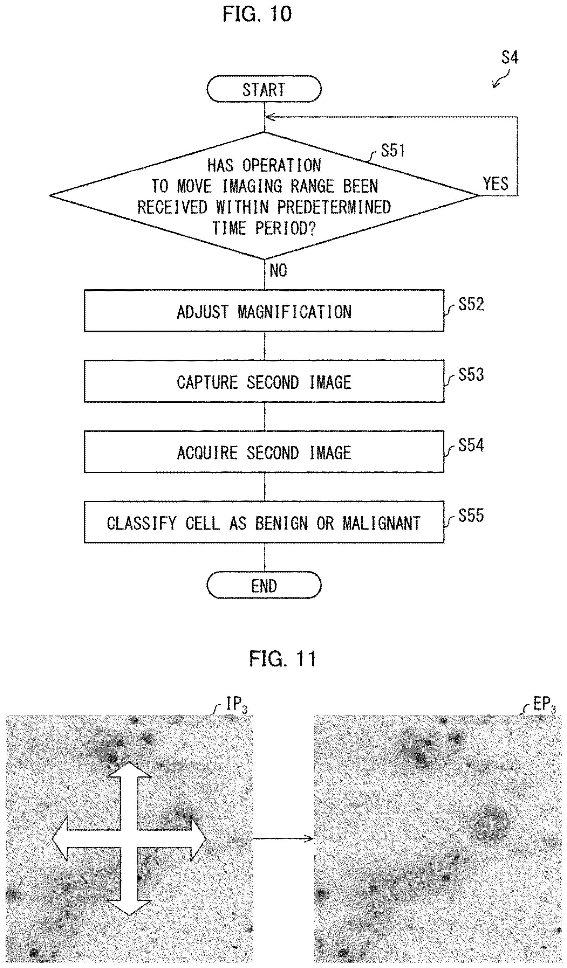

The following description will discuss a flow of a classification method S4 which is carried out by the classification apparatus 4 in accordance with the present example embodiment, with reference to Fig. 10. Fig. 10 is a flowchart illustrating the flow of the classification method S4 in accordance with the present example embodiment.

### (Step S51)

In step S51, the automatic control section 18 determines whether or not operation to move the imaging range has been received within a predetermined time period.

In a case where it has been determined in step S51 that the operation to move the imaging range has been receive within the predetermined time period (step S51: Yes), the classification apparatus 4 returns to the process of step S51.

### (Step S52)

In a case where it has been determined in step S51 that the operation to move the imaging range has not been received within the predetermined time period (step S51: No), the automatic control section 18 instructs, in step S52, the adjustment section 15 to adjust the imaging magnification from the first magnification to the second magnification. In other words, when the user moves the imaging range and a motion to search for a cell stops, the automatic control section 18 starts a process of acquiring a second image EP in the imaging range at the time of the stop.

### (Step S53)

In step S53, the imaging section 11 captures a second image EP.

### (Step S54)

In step S54, the automatic control section 18 instructs the acquisition section 12 to acquire the second image EP which has been captured by the imaging section 11. The acquisition section 12 acquires the second image EP which has been captured by the imaging section 11. The acquisition section 12 causes the storage section 208 to store the acquired second image EP.

### (Step S55)

In step S55, the automatic control section 18 instructs the classification section 13 to carry out a classification process. The classification section 13 acquires the second image EP which is stored in the storage section 20B, and classifies, as benign or malignant, a cell included as a subject in the second image EP.

### (Example 2 of displayed image)

The following description will discuss an example of an image displayed on a display apparatus in the present example embodiment, with reference to Fig. 11. Fig. 11 illustrates an example of an image which is displayed on the display apparatus in the present example embodiment.

The left part of Fig. 11 is a first image IP₃ showing a state in which the user is carrying out operation to change the imaging range. The operation by the user to change the imaging range is preferably carried out in a larger imaging range. Therefore, as illustrated in the left part of Fig. 11, the image displayed on the display apparatus is the first image IP₃ which is captured at an imaging magnification which is a first magnification lower than a second magnification.

In a case where the automatic control section 18 has determined that operation to move the imaging range has not been received within a predetermined time period while the first image IP₃ is displayed, the automatic control section 18 instructs, as illustrated in the right part of Fig. 11, the acquisition section 12 to acquire a second image EP₃ which has been captured by the imaging section 11, and causes the display apparatus to display the second image EP₃.

As described above, according to the classification apparatus 4 in accordance with the present example embodiment, the imaging range is changed by operation by the user. In a case where it has been determined that operation to change the imaging range by the user is not carried out for a predetermined time period, the classification apparatus 4 acquires a second image EP, and classifies, as benign or malignant, a cell included as a subject in the second image EP.

Therefore, the classification apparatus 4 in accordance with the present example embodiment follows operation by the user until a cell is detected, and classifies, as benign or malignant, the cell which has been found by the user. Therefore, the classification apparatus 4 in accordance with the present example embodiment can reduce labor of the user.

### (Variation)

The following description will discuss a variation of the fourth example embodiment of the present invention in detail, with reference to the drawings. The same reference numerals are given to constituent elements which have functions identical with those described in the foregoing example embodiments, and descriptions as to such constituent elements are omitted as appropriate.

In this variation, in a case where the input reception section 19 has received an instruction to cause the adjustment section 15 to adjust the imaging magnification to the second magnification, the automatic control section 18 of the classification apparatus 4 instructs the acquisition section 12 to acquire a second image EP, and instructs the classification section 13 to classify, as benign or malignant, a cell which is included as a subject in the second image EP.

### (Flow of classification method S4A)

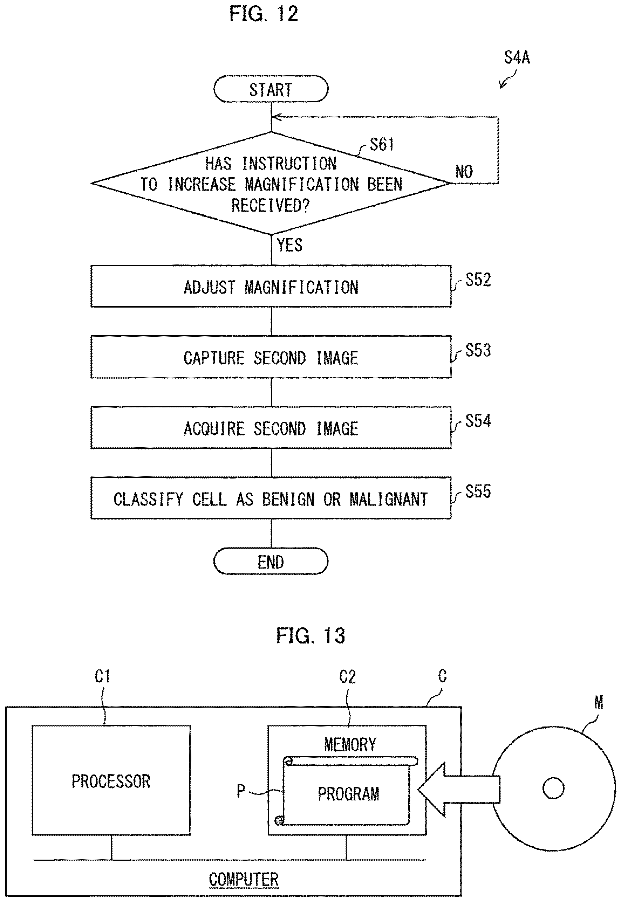

The following description will discuss a flow of a classification method S4A which is carried out by the classification apparatus 4 in accordance with this variation, with reference to Fig. 12. Fig. 12 is a flowchart illustrating a flow of the classification method S4A in accordance with this variation.

### (Step S61)

In step S61, the automatic control section 18 determines whether or not the input reception section 19 has received an instruction to increase the imaging magnification.

In step S61, in a case where it has been determined that an instruction to increase the imaging magnification has not been received (step S61: No), the classification apparatus 4 returns to the process of step S61.

### (Step S52)

In a case where it has been determined in step S61 that an instruction to increase the imaging magnification has been received (step S61: Yes), the automatic control section 18 instructs, in step S62, the adjustment section 15 to adjust the imaging magnification from the first magnification to the second magnification.

### (Steps S53 through S55)

The process in which the imaging section 11 captures a second image EP and the classification section 13 classifies, as benign or malignant, a cell included as a subject in the second image EP is as described above.

As described above, in a case where the classification apparatus 4 in accordance with this variation has received operation by the user to change the imaging magnification, the classification apparatus 4 acquires a second image EP, and classifies, as benign or malignant, a cell included as a subject in the second image EP.

Therefore, the classification apparatus 4 in accordance with this variation follows operation by the user until a cell is detected, and classifies, as benign or malignant, the cell which has been found by the user. Therefore, the classification apparatus 4 in accordance with the present example embodiment can reduce labor of the user.

### [Software implementation example]

Some or all of the functions of each of the classification apparatuses 1 through 4 may be implemented by hardware such as an integrated circuit (IC chip), or may be implemented by software.

In the latter case, the classification apparatuses 1 through 4 are implemented by, for example, a computer that executes instructions of a program that is software implementing the foregoing functions. Fig. 13 illustrates an example of such a computer (hereinafter, referred to as "computer C"). The computer C includes at least one processor C1 and at least one memory C2. The memory C2 stores a program P for causing the computer C to operate as the classification apparatuses 1 through 4. The processor C1 of the computer C retrieves the program P from the memory C2 and executes the program P, so that the functions of the classification apparatuses 1 through 4 are implemented.

As the processor C1, for example, it is possible to use a central processing unit (CPU), a graphic processing unit (CPU), a digital signal processor (DSP), a micro processing unit (MPU), a floating point number processing unit (FPU), a physics processing unit (PPU), a microcontroller, or a combination of these. Examples of the memory C2 include a flash memory, a hard disk drive (HDD), a solid state drive (SSD), and a combination thereof.

Note that the computer C can further include a random access memory (RAM) in which the program P is loaded when the program P is executed and in which various kinds of data are temporarily stored. The computer C can further include a communication interface for carrying out transmission and reception of data with other apparatuses. The computer C can further include an input-output interface for connecting input-output apparatuses such as a keyboard, a mouse, a display and a printer.

The program P can be stored in a computer C-readable, non-transitory, and tangible storage medium M. The storage medium M can be, for example, a tape, a disk, a card, a semiconductor memory, a programmable logic circuit, or the like. The computer C can obtain the program P via the storage medium M. The program P can be transmitted via a transmission medium. The transmission medium can be, for example, a communications network, a broadcast wave, or the like. The computer C can obtain the program P also via such a transmission medium.

### [Additional remark 1]

The present invention is not limited to the foregoing example embodiments, but may be altered in various ways by a skilled person within the scope of the claims. For example, the present invention also encompasses, in its technical scope, any example embodiment derived by appropriately combining technical means disclosed in the foregoing example embodiments.

### [Additional remark 2]

Some or all of the foregoing example embodiments can also be described as below. Note, however, that the present invention is not limited to the following supplementary notes.

### (Supplementary note 1)

A classification apparatus including: an imaging means for capturing an image which includes a partial range of a pathological sample as a subject; an acquisition means for acquiring the image which has been captured by the imaging means; and a classification means for classifying, as benign or malignant, a cell included as a subject in the image which has been acquired by the acquisition means.

According to the above configuration, it is possible to improve accuracy in classification of a pathological sample between benignancy and malignancy.

### (Supplementary note 2)

The classification apparatus according to supplementary note 1, further including: an adjustment means for adjusting an imaging magnification of the imaging means; a prediction means for predicting the number of cells included as a subject in the image which has been acquired by the acquisition means; and a control means for carrying out an automatic classification process in a case where (i) the adjustment means has been instructed to adjust the imaging magnification to a first magnification, (ii) the acquisition means has been instructed to acquire a first image at the first magnification, (iii) the prediction means has been instructed to predict the number of cells included as a subject in the first image, and (iv) the number of cells which has been predicted by the prediction means is equal to or greater than a predetermined number, in the automatic classification process, the adjustment means being instructed to adjust the imaging magnification to the second magnification which is higher than the first magnification, the acquisition means being instructed to acquire a second image at the second magnification, and the classification means being instructed to classify, as benign or malignant, a cell which is included as a subject in the second image.

According to the above configuration, it is possible to obtain the same classification result regardless of a skill of a user who uses the classification apparatus.

### (Supplementary note 3)

The classification apparatus according to supplementary note 2, further including: a drive means for moving an imaging range of the imaging means; in a case where the number of cells which have been classified as malignant by the classification means is less than the predetermined number as a result of the automatic classification process, the control means instructs the drive means to move the imaging range, and carries out the automatic classification process again.

According to the above configuration, it is possible to improve accuracy in classification of a pathological sample between benignancy and malignancy.

### (Supplementary note 4)

The classification apparatus according to supplementary note 3, in which: the control means instructs the drive means to exhaustively move over a whole of the pathological sample from a center of the pathological sample.

According to the above configuration, it is possible to quickly carry out a process of classifying a pathological sample between benignancy and malignancy.

### (Supplementary note 5)

The classification apparatus according to supplementary note 3 or 4, in which: the control means further carries out an automatic display process in which the drive means is instructed to move the imaging range such that a cell detected by the prediction means appears at a center of the imaging range, and a display apparatus is caused to display an image acquired by the acquisition means.

According to the above configuration, it is possible to facilitate checking work by the user.

### (Supplementary note 6)

The classification apparatus according to supplementary note 5, further including: a reception means for receiving an instruction from a user, the control means, after the automatic display process has been carried out, carrying out the automatic display process so that another cell appears at the center of the imaging range in accordance with the instruction received by the reception means.

According to the above configuration, it is possible to flexibly carry out an instruction from a user.

### (Supplementary note 7)

The classification apparatus according to supplementary note 6, in which: in a case where the instruction from the user received by the reception means is an instruction to carry out the automatic display process so that another cell appears at the center of the imaging range each time a predetermined time period elapses, the control means carries out the automatic display process so that another cell appears at the center of the imaging range each time the predetermined time period elapses.

According to the above configuration, it is possible to flexibly carry out an instruction from a user.

### (Supplementary note 8)

The classification apparatus according to supplementary note 6 or 7, in which: in a case where the instruction from the user received by the reception means is an instruction to display only a cell which has been classified as malignant, the control means carries out the automatic display process so that a cell which has been classified as malignant appears at the center of the imaging range.

According to the above configuration, it is possible to flexibly carry out an instruction from a user.

### (Supplementary note 9)

The classification apparatus according to supplementary note 1, further including: a drive means for moving an imaging range of the imaging means; an adjustment means for adjusting an imaging magnification of the imaging means; a reception means for receiving an instruction from a user; and a control means for instructing the adjustment means to adjust the imaging magnification to a first magnification or to a second magnification which is higher than the first magnification, and instructing the drive means to move the imaging range in accordance with the instruction from the user received by the reception means.

According to the above configuration, it is possible to flexibly carry out an instruction from a user.

### (Supplementary note 10)

The classification apparatus according to supplementary note 9, in which: in a case where the reception means has not received, for a predetermined time period, an instruction to cause the drive means to move the imaging range, the control means carries out an automatic classification process in which the adjustment means is instructed to adjust the imaging magnification to the second magnification which is higher than the first magnification, the acquisition means is instructed to acquire a second image at the second magnification, and the classification means is instructed to classify, as benign or malignant, a cell which is included as a subject in the second image.

According to the above configuration, it is possible to reduce labor of the user.

### (Supplementary note 11)

The classification apparatus according to supplementary note 9, wherein: in a case where the reception means has received an instruction to cause the adjustment means to adjust the imaging magnification to a second magnification which is higher than the first magnification, the control means instructs the acquisition means to acquire a second image at the second magnification, and instructs the classification means to classify, as benign or malignant, a cell which is included as a subject in the second image.

According to the above configuration, it is possible to reduce labor of the user.

### (Supplementary note 12)

A classification method including: capturing an image which includes a partial range of a pathological sample as a subject; acquiring the image which has been captured in the capturing; and classifying, as benign or malignant, a cell included as a subject in the image which has been acquired in the acquiring.

According to the above method, it is possible to improve accuracy in classification of a pathological sample between benignancy and malignancy.

### (Supplementary note 13)

A program for causing a computer to function as a classification apparatus, the program causing the computer to function as: an imaging means for capturing an image which includes a partial range of a pathological sample as a subject; an acquisition means for acquiring the image which has been captured by the imaging means; and a classification means for classifying, as benign or malignant, a cell included as a subject in the image which has been acquired by the acquisition means.

According to the above method, it is possible to improve accuracy in classification of a pathological sample between benignancy and malignancy.

### [Additional remark 3]

Some or all of the foregoing example embodiments can also be described as below.

A classification apparatus including at least one processor, the at least one processor carrying out: an imaging process of capturing an image which includes a partial range of a pathological sample as a subject; an acquisition process of acquiring the image which has been captured in the imaging process; and a classification process of classifying, as benign or malignant, a cell included as a subject in the image which has been acquired in the acquisition process.

Note that the classification apparatus can further include a memory. The memory can store a program for causing the at least one processor to execute the ... process, the ... process, and the ... process. The program can be stored in a computer-readable non-transitory tangible storage medium.

### Reference Signs List

1, 2, 3, 4: Classification apparatus
10, 10A, 10B: Control section
11: Imaging section
12: Acquisition section
13: Classification section
15: Adjustment section
16: Drive section
17: Prediction section
18: Automatic control section
19: Input reception section
20, 20A, 20B: Storage section
22: Output section
23: Input section

## Claims

1. A classification apparatus comprising:
an imaging means for capturing an image which includes a partial range of a pathological sample as a subject;
an acquisition means for acquiring the image which has been captured by the imaging means; and
a classification means for classifying, as benign or malignant, a cell included as a subject in the image which has been acquired by the acquisition means.

2. The classification apparatus according to claim 1, further comprising:
an adjustment means for adjusting an imaging magnification of the imaging means;
a prediction means for predicting the number of cells included as a subject in the image which has been acquired by the acquisition means; and
a control means for carrying out an automatic classification process in a case where (i) the adjustment means has been instructed to adjust the imaging magnification to a first magnification, (ii) the acquisition means has been instructed to acquire a first image at the first magnification, (iii) the prediction means has been instructed to predict the number of cells included as a subject in the first image, and (iv) the number of cells which has been predicted by the prediction means is equal to or greater than a predetermined number, in the automatic classification process, the adjustment means being instructed to adjust the imaging magnification to the second magnification which is higher than the first magnification, the acquisition means being instructed to acquire a second image at the second magnification, and the classification means being instructed to classify, as benign or malignant, a cell which is included as a subject in the second image.

3. The classification apparatus according to claim 2, further comprising:
a drive means for moving an imaging range of the imaging means;
in a case where the number of cells which have been classified as malignant by the classification means is less than the predetermined number as a result of the automatic classification process, the control means instructs the drive means to move the imaging range, and carries out the automatic classification process again.

4. The classification apparatus according to claim 3, wherein:
the control means instructs the drive means to exhaustively move over a whole of the pathological sample from a center of the pathological sample.

5. The classification apparatus according to claim 3 or 4, wherein:
the control means further carries out an automatic display process in which the drive means is instructed to move the imaging range such that a cell detected by the prediction means appears at a center of the imaging range, and a display apparatus is caused to display an image acquired by the acquisition means.

6. The classification apparatus according to claim 5, further comprising:
a reception means for receiving an instruction from a user,
the control means, after the automatic display process has been carried out, carrying out the automatic display process so that another cell appears at the center of the imaging range in accordance with the instruction received by the reception means.

7. The classification apparatus according to claim 6, wherein:
in a case where the instruction from the user received by the reception means is an instruction to carry out the automatic display process so that another cell appears at the center of the imaging range each time a predetermined time period elapses, the control means carries out the automatic display process so that another cell appears at the center of the imaging range each time the predetermined time period elapses.

8. The classification apparatus according to claim 6 or 7, wherein:
in a case where the instruction from the user received by the reception means is an instruction to display only a cell which has been classified as malignant, the control means carries out the automatic display process so that a cell which has been classified as malignant appears at the center of the imaging range.

9. The classification apparatus according to claim 1, further comprising:
a drive means for moving an imaging range of the imaging means;
an adjustment means for adjusting an imaging magnification of the imaging means;
a reception means for receiving an instruction from a user; and
a control means for instructing the adjustment means to adjust the imaging magnification to a first magnification or to a second magnification which is higher than the first magnification, and instructing the drive means to move the imaging range in accordance with the instruction from the user received by the reception means.

10. The classification apparatus according to claim 9, wherein:
in a case where the reception means has not received, for a predetermined time period, an instruction to cause the drive means to move the imaging range, the control means carries out an automatic classification process in which the adjustment means is instructed to adjust the imaging magnification to the second magnification which is higher than the first magnification, the acquisition means is instructed to acquire a second image at the second magnification, and the classification means is instructed to classify, as benign or malignant, a cell which is included as a subject in the second image.

11. The classification apparatus according to claim 9, wherein:
in a case where the reception means has received an instruction to cause the adjustment means to adjust the imaging magnification to a second magnification which is higher than the first magnification, the control means instructs the acquisition means to acquire a second image at the second magnification, and instructs the classification means to classify, as benign or malignant, a cell which is included as a subject in the second image.

12. A classification method comprising:
capturing an image which includes a partial range of a pathological sample as a subject;
acquiring the image which has been captured in the capturing; and
classifying, as benign or malignant, a cell included as a subject in the image which has been acquired in the acquiring.

13. A program for causing a computer to function as a classification apparatus, the program causing the computer to function as:
an imaging means for capturing an image which includes a partial range of a pathological sample as a subject;
an acquisition means for acquiring the image which has been captured by the imaging means; and
a classification means for classifying, as benign or malignant, a cell included as a subject in the image which has been acquired by the acquisition means.
